(19) **Europäisches Patentamt European Patent Office Office européen des brevets**

(11) **EP 4 474 817 B1**

(12) **FASCICULE DE BREVET EUROPEEN**

(45) Date de publication et mention de la délivrance du brevet:
**13.05.2026 Bulletin 2026/20**

(21) Numéro de dépôt: **24180563.9**

(22) Date de dépôt: **06.06.2024**

(51) Classification Internationale des Brevets (IPC):
**B01D 53/26** *(2006.01)*      **C01B 3/06** *(2026.01)*
**C01B 3/10** *(2026.01)*      **E21B 43/24** *(2006.01)*
**G01N 33/24** *(2006.01)*

(52) Classification Coopérative des Brevets (CPC):
**G01N 33/24; C01B 3/061; C01B 3/10; E21B 43/24;**
B01D 53/265

(54) **PROCÉDÉ DE QUANTIFICATION DU POTENTIEL DE GÉNÉRATION D'HYDROGÈNE NATUREL ISSU D'ECHANTILLON DE ROCHES**

VERFAHREN ZUR QUANTIFIZIERUNG DES PRODUKTIONSPOTENTIALS VON NATÜRLICHEM WASSERSTOFF AUS EINER GESTEINSPROBE

METHOD FOR QUANTIFYING THE GENERATION POTENTIAL OF NATURAL HYDROGEN FROM ROCK SAMPLE

(84) Etats contractants désignés:
**AL AT BE BG CH CY CZ DE DK EE ES FI FR GB GR HR HU IE IS IT LI LT LU LV MC ME MK MT NL NO PL PT RO RS SE SI SK SM TR**

(30) Priorité: **09.06.2023 FR 2305855**

(43) Date de publication de la demande:
**11.12.2024 Bulletin 2024/50**

(73) Titulaire: **Vinci Technologies**
**92000 Nanterre (FR)**

(72) Inventeurs:
• **AMMOUIAL, Jérémie**
**92100 Boulogne-Billancourt (FR)**
• **BOUTON, Nicolas**
**94800 Villejuif (FR)**

(74) Mandataire: **Alatis**
**3, rue Paul Escudier**
**75009 Paris (FR)**

(56) Documents cités:
US-A- 3 442 620      US-A- 3 880 987
US-A- 4 078 904      US-A1- 2016 039 669
US-A1- 2023 102 312

**Description**

La présente invention concerne un procédé et un dispositif d'analyse en continu d'un flux d'hydrogène généré par interaction chimique entre un gaz vecteur chargé en eau et un échantillon de roche minérale.

De manière générale, l'invention vise à déterminer la quantité d'hydrogène générée par un échantillon de roche lors d'une réaction de réduction de l'eau à haute température et en présence de cet échantillon. L'invention vise aussi à analyser la cinétique de la réaction de réduction en fonction de la température et de la quantité d'eau.

Du fait du déclin des ressources en hydrocarbures et des besoins énergétiques mondiaux croissants, et de la recherche d'un mix énergétique moins carboné la production d'hydrogène dans le monde et son utilisation sont devenus des enjeux majeurs.

Au su d'être une matière première pour l'industrie chimique, en particulier pour la fabrication d'engrais, l'hydrogène est un carburant. Les faibles émissions de gaz à effet de serre produites par la combustion de l'hydrogène en font un bon candidat comme ressource respectueuse de l'environnement dans un contexte de réchauffement climatique.

Cependant, la production actuelle d'hydrogène reste très dépendante des énergies fossiles. En effet, environ 60% de l'hydrogène produit est issu du vapo-reformage du méthane (ou gaz naturel) à haute température elle-même produite le plus souvent via des énergies fossiles. Environ 20% vient de la gazéification du charbon et le reste des HC liquides. Moins de 5% sont issus de l'électrolyse, procédé très gourmand en énergie électrique alors que celle-ci est produite le plus souvent par des centrales thermiques à hydrocarbures ou à charbon.

La principale problématique que pose l'hydrogène en tant qu'énergie propre, ou matière première, est donc sa production. Dès lors l'exploration de sources d'hydrogène naturel prend tout son sens. Bien que toujours à l'état prospectif, la compréhension des systèmes naturels de génération de di-hydrogène devrait permettre à l'avenir d'exploiter cet hydrogène naturel sans recourir à des énergies fossiles. C'est dans ce contexte que l'invention apporte des solutions pour simuler en laboratoire les conditions naturelles de production d'hydrogène dans les couches géologiques.

Les applications visées par l'invention sont principalement situées dans le domaine de l'exploration des sources d'hydrogène naturel à travers le monde. Plus précisément, l'invention s'intéresse principalement (mais non exclusivement) à un mode particulier de génération d'hydrogène à partir de roches minérales. Ce mode de génération résulte de l'interaction entre l'eau, présente en abondance dans tous les milieux géologiques profonds, et les minéraux ferreux présents dans certains types de roches. Cette génération s'effectue à l'état naturel dans différents milieux géologiques particuliers, comme dans les rides médio océaniques, les zones d'obduction, les zones de subduction et dans certains cratons archéens et Néoprotérozoïques. Ces milieux sont soumis à des températures élevées allant de 100°C à 400°C (et parfois plus) favorisant ainsi une réaction oxydo-réductrice.

Plusieurs réactions chimiques permettent de générer de l'hydrogène naturellement. La plus connue est la réaction de serpentinisation qui caractérise l'hydratation de l'olivine en serpentine. Cette réaction génère de l'hydrogène comme sous-produit. Plus précisément, l'eau entraine une oxydation du fer contenu dans la fayalite (pôle ferrugineux de l'olivine représentant environ 10% des olivines) selon la réaction suivante :

$$2H_2O + 3Fe_2SiO_4 = 2Fe_3O_4 + 3SiO_2 + 2H_{2\,(g)}$$

Une réaction similaire susceptible de produire de l'hydrogène existe également lors de l'hydratation de l'orthopyroxène. Dans les cratons, ce sont les roches riches en fer, en particulier les BIF (Banded Iron Formation) qui vont s'oxyder, le fer passant de ferreux ($Fe^{2+}$) à ferrique ($Fe^{3+}$). Cette même réaction peut avoir lieu en présence d'amphiboles, présentes dans les granites, elles sont elles aussi riches en fer. US 2016039669A1 divulgue un tel procédé de production d'hydrogène.

Tout comme lors des prémices de l'exploration pétrolière, les premiers indices d'un phénomène de génération d'hydrogène dans le sous-sol ont étés trouvés en surface. En effet, certains endroits dans le monde sont connus pour leurs émanations de di-hydrogène naturel, comme les feux de Yanartas en Turquie qui brûlent depuis plus de 2500 ans. D'autres émanations d'hydrogène ont également été repérées en surface associées à une géomorphologie singulière de type dépression circulaire mesurant de quelques mètres à plusieurs kilomètres appelés « ronds de sorcières » et dans lesquelles la végétation est différente.

Par ailleurs, un gisement d'hydrogène naturel « onshore » a été découvert par hasard et est en cours d'exploitation au Mali par la société Petroma (désormais Hydroma) depuis 2010. Ce puits de faible profondeur permet l'alimentation d'une turbine qui génère de l'électricité pour un village de 1500 habitants. Depuis, de nombreux permis d'explorations ont été demandés dans le monde comme aux Etats-Unis, en France et en Australie.

Côté « offshore » certains environnements géologiques ont été identifiés comme pouvant générer de l'hydrogène naturel. C'est le cas de toutes les rides médio océaniques mais aussi de la fosse des Mariannes dans laquelle plusieurs volcans de boues serpentinisés produisent un gaz mélangeant hydrogène et méthane.

Dans ce contexte, l'invention consiste en un procédé et un dispositif capables d'analyser rapidement et automatiquement en laboratoire des échantillons de roches en vue de déterminer la quantité d'hydrogène générée au cours d'une réaction de réduction de l'eau en présence de cet échantillon. L'invention permet ainsi de déduire le potentiel rémanent de production d'hydrogène naturel à partir des roches d'où est issu cet échantillon.

Ce but est atteint selon un premier aspect de l'invention au moyen d'un procédé de quantification automatique du dihydrogène généré par un échantillon de roche mère minérale contenant un composé métallique ferreux, caractérisé en ce qu'on effectue une réaction de réduction de l'eau en présence dudit échantillon et de vapeur d'eau de 300°C jusqu'à une température d'environ 1200°C et on détermine la quantité dudit dihydrogène généré par ledit échantillon lors de cette réaction en vue de qualifier ladite roche mère comme source potentielle d'hydrogène naturel.

Un des paramètres recherchés par le procédé selon l'invention est la température (TmH2) correspondant à la génération d'une quantité maximale d'hydrogène et permettant de déterminer la cinétique de génération dudit hydrogène.

Un autre paramètre recherché par le procédé de l'invention est le rendement molaire ($\eta_n$H2) et/ou le rendement massique ($\eta_m$H2) de la réaction de réduction de l'eau caractérisé par le rapport entre la quantité molaire d'hydrogène produite (nH2) et la quantité molaire de l'échantillon de roche contenant le fer (n réactif) ou sa masse dans le cas du rendement massique, et qui est défini selon les formules suivantes :

$$\eta_n(H2) = \frac{nH2\ prod}{n\ réactif} \times 100 \quad \text{et} \quad \eta_m(H2) = \frac{mH2\ prod}{m\ réactif} \times 100$$

Le procédé de l'invention utilise ainsi la quantité d'hydrogène générée par rapport à la masse d'échantillon utilisé lors de l'analyse pour calculer les rendements molaire et massique utiles ($N_n$H2 et $N_m$H2) et déterminer le potentiel hydrogène ainsi que la maturité de la roche mère.

Selon une caractéristique avantageuse de mise en œuvre de ce procédé, l'eau est mise en présence de l'échantillon de roche au moyen d'un gaz vecteur inerte transportant de la vapeur d'eau.

La température initiale du réacteur est comprise entre 200°C et 400°C et la température finale de la réaction de réduction est comprise entre 600°C et 1200°C.

De préférence, le gradient de l'élévation de température est compris entre 0,1 à 50°C/min.

Le débit du gaz vecteur est compris entre 0 et 500mL/min et sa teneur en eau est contrôlée via l'élévation de température d'une réserve d'eau liquide ou par vaporisation d'eau liquide dans un flux de gaz chaud.

A la sortie du réacteur, les effluents gazeux issus de la réaction de réduction de l'eau sont séparés pour assurer la condensation de l'eau et le séchage de la fraction gazeuse des effluents.

Ensuite, cette fraction gazeuse est analysée en vue de déterminer la quantité d'hydrogène produite par l'échantillon.

Un autre objet de l'invention est un dispositif pour la mise en œuvre du procédé décrit ci-dessus de quantification en continu de l'hydrogène émis par un échantillon de roche mère minérale ou sédimentaire, caractérisé en ce qu'il comprend un réacteur destiné à recevoir ledit échantillon, ledit réacteur étant pourvu d'une tubulure d'injection d'un gaz vecteur alimentée par un ballon contenant de l'eau chauffée entre 0 et 100°C et pourvue d'une ligne de maintien en température, des moyens de chauffage assurant des montées en température de 0 à 1200°C à l'intérieur du réacteur, un séparateur collectant les effluents gazeux issus du réacteur et assurant la condensation de l'eau et l'échappement de la fraction gazeuse des effluents vers un moyen de séchage et un détecteur d'hydrogène analysant la fraction gazeuse séchée.

Selon une caractéristique avantageuse de l'invention, le dispositif comprend un piston assurant l'introduction de l'échantillon dans le réacteur.

En outre, le dispositif comprend des thermocouples destinés à contrôler les températures du ballon et de la ligne de maintien en température du gaz vecteur injecté ainsi qu'un débitmètre assurant le contrôle du débit du gaz vecteur injecté.

De préférence, le dispositif de l'invention comprend une buse d'injection pilotant la quantité d'eau injectée dans le réacteur ainsi qu'un hygromètre ou un détecteur de point de rosé destiné à contrôler la teneur en eau du gaz vecteur injecté.

L'invention permet d'apporter une information précise et fiable aux entreprises de prospection de l'hydrogène d'origine naturelle à partir de simples échantillons de roches mères prélevées dans le sous-sol. En particulier, cette information assure une bonne visibilité quant au potentiel d'une zone géologique pour la production d'hydrogène.

La mise en œuvre du procédé de quantification peut être effectuée en laboratoire au moyen d'un dispositif simple et facile à mettre en œuvre, y compris de façon nomade et/ou délocalisée.

L'invention permet ainsi l'analyse du potentiel de génération de l'hydrogène d'un échantillon de roche en moins d'une heure tandis que des analyses similaires en autoclaves, par exemple, peuvent durer des semaines.

D'autres caractéristiques et avantages de l'invention ressortiront à la lecture de description qui va suivre, en référence aux figures annexées qui sont détaillées ci-après.

[Fig. 1] représente un mode de réalisation du dispositif de l'invention avec le cheminement du gaz vecteur au cours de la mise en œuvre du procédé.

[Fig. 2] est un graphique représentant la génération d'hydrogène en fonction de la température à partir d'un échantillon de sidérite.

[Fig. 3] est un graphique représentant la génération d'hydrogène en fonction de la température à partir d'un échantillon d'olivine.

Pour plus de clarté, les éléments identiques ou similaires sont repérés par des signes de référence identiques dans la description et sur les figures.

Naturellement, les modes de mise en œuvre du procédé de l'invention et de réalisation du dispositif associé illustrés schématiquement par les figures présentées ci-dessus et décrites ci-après, ne sont donnés qu'à titre d'exemple non limitatif. Il est explicitement prévu dans le cadre de l'invention que l'on puisse proposer et combiner entre eux différents modes pour en proposer d'autres.

De manière générale, l'invention concerne le domaine de la prospection de sources d'hydrogène naturel via l'analyse de roches mères à hydrogène en laboratoire. Plus précisément, le procédé de l'invention consiste à quantifier, de façon automatique, le dihydrogène généré par un échantillon de roche minérale ou sédimentaire contenant un composé métallique ferreux lors d'une réaction de réduction de l'eau jusqu'à une température de 1200°C. La détermination de la quantité du dihydrogène généré par cet échantillon permet ainsi la qualification de la roche mère comme source potentielle d'hydrogène naturel via les paramètres ηH2 et TmH2.

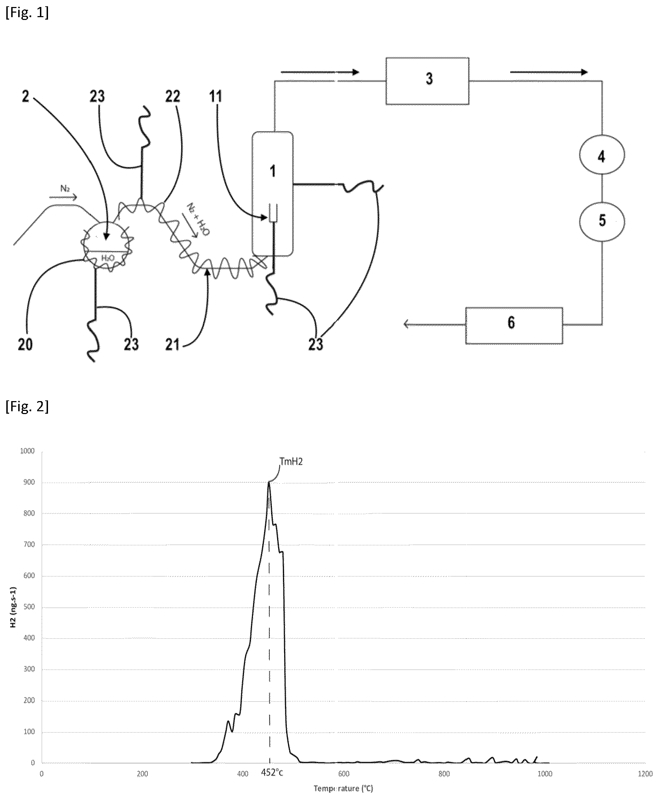

A cette fin, de l'eau sous forme vapeur est mise en présence de l'échantillon de roche dans un réacteur 1 au moyen d'un gaz vecteur inerte (par exemple de l'azote $N_2$), comme illustré par la figure 1. Les effluents gazeux issus de la réaction de réduction de l'eau à haute température sont acheminés et séparés pour assurer la condensation de l'eau et le séchage de la fraction gazeuse. Cette fraction gazeuse séchée est ensuite analysée à l'issue du procédé pour déterminer la quantité d'hydrogène présente initialement dans l'échantillon.

La température initiale de l'eau sous forme vapeur injectée dans le réacteur 1 est comprise entre 200°C et 400°C et la température finale de la réaction de réduction est comprise entre 600°C et 1200°C.

De préférence, le gradient de l'élévation de température dans le réacteur 1 est compris entre 0,1 à 50°C/min afin de pouvoir étudier la cinétique de génération de l'hydrogène. Le débit du gaz vecteur injecté dans le réacteur est compris entre 0 et 500mL/min et la teneur en eau du gaz vecteur est contrôlée via l'élévation en température d'une réserve d'eau liquide.

De préférence, le cycle de température intégré au procédé débute à 300°C et présente un palier de 3 min. Puis, une élévation de température est effectuée à un taux de 25°C/min jusqu'à une température finale de 1200°C.

Un mode de réalisation du dispositif pour la mise en œuvre de ce procédé est représenté sur la figure 1. Ce dispositif comprend un réacteur céramique 1 alimenté par une tubulure 21 assurant l'injection en continu d'un gaz vecteur inerte. Ce réacteur est conçu pour pouvoir résister à des températures allant jusqu'à 1200°C obtenues grâce à un bobinage de fils chauffant dont les spires ont étés judicieusement réparties afin d'obtenir une température homogène et précise au niveau de l'échantillon. Le ou les échantillons de roches sont introduits dans le réacteur 1 via un système de passeur automatique permettant de déposer les échantillons sur un piston 11 assurant leur déplacement en translation dans le réacteur 1 et son étanchéité une fois fermé.

Le gaz vecteur est préalablement chargé en eau au moyen d'un système d'hydratation/humidification. A cet effet, la tubulure d'injection 21 du gaz vecteur est raccordée à un ballon 2 contenant de l'eau chauffée entre 0 et 100°C. Ainsi, selon le mode de mise en œuvre du procédé et au moyen du dispositif illustré par la figure 1, le gaz vecteur injecté dans le réacteur 1 permet d'entraîner une quantité d'eau qui est destinée à réagir dans le réacteur 1 avec la phase minérale de l'échantillon de roche.

Le système d'hydratation du gaz vecteur comprend ici un ballon 2 contenant de l'eau distillée qui est disposé en amont du réacteur 1 de pyrolyse et dans lequel le gaz vecteur vient buller. Un dispositif de contrôle de l'hygrométrie du gaz pourra également être ajouté optionnellement afin de tester différents taux d'humidifications. Ce ballon 2 est équipé de moyens thermiques 20 (telles que des résistances à effet joule) assurant le chauffage de l'eau de 0 à 100°C de façon à assurer son passage de la phase liquide à la phase vapeur.

La tubulure 21 de gaz allant du ballon 2 à l'entrée du réacteur 1 est elle-même maintenue à une température appropriée à l'aide d'une ligne 22 assurant le maintien en température et évitant ainsi toute condensation avant l'interaction chimique avec l'échantillon de roche. Le dispositif de l'invention comprend, en outre, un système d'électrovannes (non représentées) dont l'activation automatique permet de choisir le by-pass du mode d'humidification.

Le débit du gaz vecteur pourra être contrôlé à l'aide de débitmètres massiques (non représentés) pouvant effectuer des variations de débits allant de 0 à 500 mL/min.

En outre, le dispositif comprend des thermocouples 23 destinés à contrôler les températures du ballon 2, de la tubulure 21 du gaz vecteur injecté et du réacteur 1 (situé en dessous de l'échantillon et sur la paroi dudit réacteur) ainsi qu'un débitmètre (non représenté) assurant le contrôle du débit du gaz vecteur injecté.

De préférence, le dispositif de l'invention comprend une buse d'injection (non représentée) pilotant les paramètres du gaz vecteur ainsi qu'un hygromètre et/ou un détecteur de point de rosé (non représentés) destinés à contrôler la teneur en eau du gaz vecteur injecté.

En sortie du réacteur 1, le dispositif comprend un système 3 assurant la séparation entre les effluents gazeux issus de l'échantillon pyrolysé et la fraction liquide. Ces effluents passent ensuite au travers d'un moyen de séchage tel qu'un dessicant 4 afin d'éliminer toute présence d'eau résiduelle.

Un détecteur 6 d'hydrogène assure l'analyse finale de la fraction gazeuse séchée et permet de déterminer la quantité d'hydrogène présente initialement dans l'échantillon. Selon le type de détecteur d'hydrogène 6 utilisé en fin de ligne, il sera possible d'ajouter une série de pièges 5 afin d'en retirer les éléments susceptibles d'interférer avec la mesure.

Des exemples de mise en œuvre du procédé de l'invention vont maintenant être décrits en détails ci-après en étant appliqués à deux échantillons de roches différents mais dans des conditions expérimentales identiques.

Dans ces exemples, la température du ballon 2 est maintenue à 90°C ainsi que la tubulure d'injection 21. Le gaz vecteur circulant dans le ballon d'eau a un débit de 100 mL/min. Le cycle de température utilisé démarre à 300°C avec un palier de température de 3min et se termine à 1000°C. Le gradient de température utilisé est de 20°C/min.

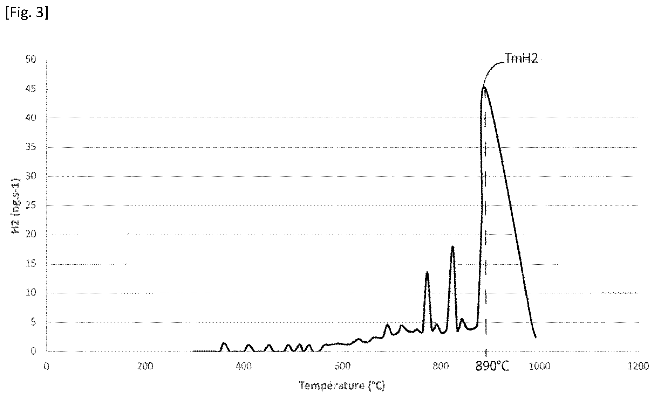

Les deux échantillons analysés sont, respectivement, de la sidérite (carbonate de fer - figure 2) et de l'olivine (principal constituant minéral du manteau terrestre - figure 3). La sidérite, de formule $FeCO_3$, est d'origine synthétique et pure à 100% avec une teneur en fer de l'ordre de 48%. L'olivine est de type forstérite naturelle avec une teneur en fer d'environ 3,8%.

Lors de l'analyse de 120 mg de sidérite, un important pic d'hydrogène est généré entre 350°C et 500°C correspondant à la plage de décarbonatation de ce minéral (figure 2). L'intégration de cette courbe permet d'évaluer la production à environ 0,16 mg, soit 0,08 moles d'hydrogène pour 1 mole de sidérite analysée, représentant un rendement de 8%. Puis la production d'hydrogène retombe à zéro par la suite. Dans cet exemple, la température (TmH2) est égale à 452°C.

L'analyse de 120mg de forstérite produit moins d'hydrogène, ce qui est prévisible car cette roche contient douze fois moins de fer que la sidérite. Le rendement molaire est cette fois-ci de 0,6%. L'hydrogène commence à être produit à partir de 600°C mais c'est entre 800°C et 1000°C que le pic de production est observé (figure 3). Dans cet exemple, la température (TmH2) est égale à 890°C.

### Revendications

1. Procédé de quantification automatique du dihydrogène généré par un échantillon de roche mère minérale ou sédimentaire contenant un composé métallique contenant du fer, dans lequel on effectue une réaction de réduction de l'eau en présence dudit échantillon et de vapeur d'eau dont la température est montée jusqu'à environ 1200°C et on détermine la quantité dudit dihydrogène généré par ledit échantillon lors de cette réaction en vue de qualifier ladite roche mère comme source potentielle d'hydrogène naturel.

2. Procédé selon la revendication 1, **caractérisé en ce que** l'eau est mise en présence de l'échantillon de roche au moyen d'un gaz vecteur inerte transportant de la vapeur d'eau.

3. Procédé selon l'une des revendications précédentes, **caractérisé en ce que** les effluents gazeux issus de la réaction de réduction de l'eau sont séparés pour assurer la condensation de l'eau et le séchage de la fraction gazeuse desdits effluents.

4. Procédé selon la revendication précédente, **caractérisé en ce que** la fraction gazeuse séchée est analysée en vue de déterminer la quantité d'hydrogène présente initialement dans l'échantillon.

5. Procédé selon l'une des revendications précédentes, **caractérisé en ce que** la température initiale de la vapeur d'eau avant la montée en température est comprise entre 200°C et 400°C.

6. Procédé selon l'une des revendications précédentes **caractérisé en ce que** la température finale de la vapeur d'eau à l'issue de la montée en température lors de la réaction de réduction est comprise entre 600°C et 1200°C.

7. Procédé selon l'une des revendications précédentes, **caractérisé en ce que** le gradient de l'élévation de température est compris entre 0,1 à 50°C/min.

8. Procédé selon l'une des revendications précédentes, **caractérisé en ce que** le débit du gaz vecteur est compris entre 0 et 500mL/min.

9. Procédé selon l'une des revendications précédentes, **caractérisé en ce que** la teneur en eau du gaz vecteur est contrôlée via l'élévation en température d'une réserve d'eau ou par vaporisation d'eau liquide dans un flux de gaz chaud.

10. Procédé selon l'une des revendications précédentes, **caractérisé en ce qu'**on détermine la température (TmH2) correspondant à la génération d'une quantité maximale d'hydrogène et on en déduit la cinétique de génération dudit hydrogène.

11. Procédé selon l'une des revendications précédentes, **caractérisé en ce qu'**on utilise la quantité d'hydrogène générée par l'échantillon pour calculer les rendements molaire et massique utiles ($N_nH2$ et $\eta_mH2$) et déterminer la quantité potentielle d'hydrogène de la roche mère ainsi que la maturité de la roche mère dont est issu ledit échantillon.

12. Dispositif pour la quantification en continu de l'hydrogène émis par un échantillon de roche mère minérale ou sédimentaire, **caractérisé en ce qu'**il comprend un réacteur (1) destiné à recevoir ledit échantillon, ledit réacteur étant pourvu d'une tubulure (21) d'injection d'un gaz vecteur alimentée par un ballon (2) contenant de l'eau chauffée entre 0 et 100°C et pourvue d'une ligne (22) de maintien en température, un réacteur avec bobinage de fils chauffant assurant des montées en température de 0 à 1200°C à l'intérieur dudit réacteur, un séparateur (3) collectant les effluents gazeux issus du réacteur et assurant la condensation de l'eau et l'échappement de la fraction gazeuse des effluents vers un moyen de séchage (4) et un détecteur (6) d'hydrogène analysant la fraction gazeuse séchée.

13. Dispositif selon la revendication 12, **caractérisé en ce qu'**il comprend un piston (11) assurant l'introduction de l'échantillon dans le réacteur (1).

14. Dispositif selon l'une des revendication 12 ou 13, **caractérisé en ce qu'**il comprend des thermocouples (23) destinés à contrôler les températures du ballon (2) et de la ligne (22) de maintien en température du gaz vecteur injecté.

15. Dispositif selon l'une des revendication 12 à 14, **caractérisé en ce qu'**il comprend un débitmètre assurant le contrôle du débit du gaz vecteur injecté.

16. Dispositif selon l'une des revendications 12 à 15, **caractérisé en ce qu'**il comprend une buse d'injection pilotant la quantité d'eau injectée dans le réacteur (1).

17. Dispositif selon l'une des revendications 12 à 16, **caractérisé en ce qu'**il comprend un hygromètre ou un détecteur de point de rosé destiné à contrôler la teneur en eau du gaz vecteur injecté.

**Patentansprüche**

1. Verfahren zur automatischen Quantifizierung des von einer mineralischen oder sedimentären Muttergesteinsprobe erzeugten Diwasserstoffs, die eine eisenhaltige Metallverbindung enthält, wobei eine Reduktionsreaktion von Wasser in Gegenwart der Probe und von Wasserdampf durchgeführt wird, dessen Temperatur auf etwa 1.200 °C erhöht wird, und die Menge des von der Probe bei dieser Reaktion erzeugten Diwasserstoffs bestimmt wird, um das Muttergestein als potentielle Quelle für natürlichen Wasserstoff zu qualifizieren.

2. Verfahren nach Anspruch 1, **dadurch gekennzeichnet, dass** das Wasser mittels eines inerten Trägergases, das Wasserdampf transportiert, mit der Gesteinsprobe in Kontakt gebracht wird.

3. Verfahren nach einem der vorstehenden Ansprüche,
**dadurch gekennzeichnet, dass** die aus der Wasserreduktionsreaktion stammenden gasförmigen Abwässer getrennt werden, um die Kondensation des Wassers und die Trocknung des gasförmigen Anteils der Abwässer zu gewährleisten.

4. Verfahren nach dem vorstehenden Anspruch,
**dadurch gekennzeichnet, dass** der getrocknete Gasanteil analysiert wird, um die ursprünglich in der Probe vorhandene Menge an Wasserstoff zu bestimmen.

5. Verfahren nach einem der vorstehenden Ansprüche,
**dadurch gekennzeichnet, dass** die Anfangstemperatur des Wasserdampfs vor dem Temperaturanstieg zwischen 200 °C und 400 °C liegt.

**6.** Verfahren nach einem der vorstehenden Ansprüche,
**dadurch gekennzeichnet, dass** die Endtemperatur des Wasserdampfes am Ende des Temperaturanstiegs während der Reduktionsreaktion zwischen 600 °C und 1.200 °C liegt.

**7.** Verfahren nach einem der vorstehenden Ansprüche,
**dadurch gekennzeichnet, dass** der Gradient der Temperaturerhöhung zwischen 0,1 und 50 °C/Min. liegt.

**8.** Verfahren nach einem der vorstehenden Ansprüche,
**dadurch gekennzeichnet, dass** die Durchflussrate des Trägergases zwischen 0 und 500 ml/Min. liegt.

**9.** Verfahren nach einem der vorstehenden Ansprüche,
**dadurch gekennzeichnet, dass** der Wassergehalt des Trägergases über die Temperaturerhöhung eines Wasservorrats oder durch Verdampfung von flüssigem Wasser in einem heißen Gasstrom kontrolliert wird.

**10.** Verfahren nach einem der vorstehenden Ansprüche,
**dadurch gekennzeichnet, dass** die Temperatur (TmH2) bestimmt wird, die der Erzeugung einer maximalen Menge an Wasserstoff entspricht, und daraus die Kinetik der Erzeugung des Wasserstoffs ableitet.

**11.** Verfahren nach einem der vorstehenden Ansprüche,
**dadurch gekennzeichnet, dass** die von der Probe erzeugte Wasserstoffmenge verwendet wird, um die nützlichen molaren und Massenausbeuten ($\eta_n$H2 und $\eta_m$H2) zu berechnen und die potentielle Wasserstoffmenge des Muttergesteins sowie die Reife des Muttergesteins, aus dem die Probe stammt, zu bestimmen.

**12.** Vorrichtung zur kontinuierlichen Quantifizierung des von einer mineralischen oder sedimentären Muttergesteinsprobe emittierten Wasserstoffs, **dadurch gekennzeichnet, dass** sie einen Reaktor (1) umfasst, der zur Aufnahme der Probe bestimmt ist, wobei der Reaktor mit einer Rohrleitung (21) zur Einspritzung eines Trägergases versehen ist, die von einem Ballon (2) gespeist wird, der zwischen 0 und 100 °C erwärmtes Wasser enthält und mit einer Temperaturhalteleitung (22) versehen ist, einen Reaktor mit Heizdrähtewicklung, die Temperaturanstiege von 0 bis 1.200 °C im Inneren des Reaktors gewährleistet, einen Abscheider (3), der die aus dem Reaktor stammenden gasförmigen Abwässer sammelt und die Kondensation des Wassers sowie das Entweichen des gasförmigen Anteils der Abwässer zu einem Trocknungsmittel (4) gewährleistet, und einen Wasserstoffdetektor (6), der den getrockneten gasförmigen Anteil analysiert.

**13.** Vorrichtung nach Anspruch 12, **dadurch gekennzeichnet, dass** sie einen Kolben (11) umfasst, der die Einführung der Probe in den Reaktor (1) gewährleistet.

**14.** Vorrichtung nach einem der Ansprüche 12 oder 13,
**dadurch gekennzeichnet, dass** sie Thermoelemente (23) umfasst, die dazu bestimmt sind, die Temperaturen des Ballons (2) und der Leitung (22) zur Temperaturhaltung des injizierten Trägergases zu kontrollieren.

**15.** Vorrichtung nach einem der Ansprüche 12 bis 14,
**dadurch gekennzeichnet, dass** sie einen Durchflussmesser umfasst, der die Kontrolle des Durchflusses des eingespritzten Trägergases gewährleistet.

**16.** Vorrichtung nach einem der Ansprüche 12 bis 15,
**dadurch gekennzeichnet, dass** sie eine Einspritzdüse umfasst, die die in den Reaktor (1) eingespritzte Wassermenge steuert.

**17.** Vorrichtung nach einem der Ansprüche 12 bis 16,
**dadurch gekennzeichnet, dass** sie ein Hy grometer oder einen Taupunktdetektor umfasst, der dazu bestimmt ist, den Wassergehalt des eingespritzten Trägergases zu kontrollieren.

**Claims**

**1.** Method for automatically quantifying the dihydrogen generated by a sample of mineral or sedimentary parent rock containing an iron-containing metal compound, wherein a water reduction reaction is carried out in the presence of said sample and water vapor, the temperature of which is rised to approximately 1200 °C, and the amount of said dihydrogen generated by said sample during this reaction is determined in order to qualify said parent rock as a potential source of natural hydrogen.

2. Method according to claim 1, **characterized in that** the water is brought into contact with the rock sample by means of an inert carrier gas carrying water vapor.

3. Method according to either of the preceding claims, **characterized in that** the gaseous effluents from the water reduction reaction are separated to ensure condensation of the water and drying of the gaseous fraction of said effluents.

4. Method according to the preceding claim, **characterized in that** the dried gaseous fraction is analyzed in order to determine the amount of hydrogen initially present in the sample.

5. Method according to any one of the preceding claims, **characterized in that** the initial temperature of the water vapor prior to the temperature rise is between 200 °C and 400 °C.

6. Method according to any one of the preceding claims,
**characterized in that** the final temperature of the water vapor at the end of the temperature rise during the reduction reaction is between 600 °C and 1200 °C.

7. Method according to any one of the preceding claims, **characterized in that** the gradient of the temperature increase is between 0.1 and 50 °C/min.

8. Method according to any one of the preceding claims, **characterized in that** the carrier gas flow rate is between 0 and 500 mL/min.

9. Method according to any one of the preceding claims, **characterized in that** the water content of the carrier gas is monitored by increasing the temperature of a water reserve or by vaporizing liquid water in a hot gas flow.

10. Method according to any one of the preceding claims, **characterized in that** the temperature (TmH2) corresponding to the generation of a maximum amount of hydrogen is determined, and the kinetics of the generation of said hydrogen is deduced therefrom.

11. Method according to any one of the preceding claims, **characterized in that** the quantity of hydrogen generated by the sample is used to calculate the useful molar and mass yields ($\eta_n H2$ and $\eta_m H2$) and to determine the potential amount of hydrogen in the parent rock, as well as the maturity of the parent rock from which said sample was taken.

12. Device for continuously quantifying the hydrogen emitted by a sample of mineral or sedimentary parent rock, **characterized in that** it comprises a reactor (1) intended to receive said sample, said reactor being provided with a carrier gas injection tube (21) fed by a tank (2) containing water heated to between 0 and 100 °C and provided with a line (22) for maintaining the temperature, a reactor with a winding of heating wires ensuring temperature rises from 0 to 1200 °C inside said reactor, a separator (3) collecting the gaseous effluents from the reactor and ensuring the condensation of the water and exhaust of the gaseous fraction of the effluents to a drying means (4) and a hydrogen detector (6) analyzing the dried gaseous fraction.

13. Device according to claim 12, **characterized in that** it comprises a piston (11) ensuring the introduction of the sample into the reactor (1).

14. Device according to any one of claims 12 or 13, **characterized in that** it comprises thermocouples (23) intended for monitoring the temperatures of the tank (2) and the line (22) for maintaining the temperature of the injected carrier gas.

15. Device according to any one of claims 12 to 14, **characterized in that** it comprises a flowmeter for monitoring the flow rate of the injected carrier gas.

16. Device according to any one of claims 12 to 15, **characterized in that** it comprises an injection nozzle controlling the amount of water injected into the reactor (1).

17. Device according to any one of claims 12 to 16, **characterized in that** it comprises a hygrometer or dew point detector intended for monitoring the water content of the injected carrier gas.

[Fig. 1]

[Fig. 2]

[Fig. 3]

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- US 2016039669 A1